# EUROPEAN PATENT SPECIFICATION

(11) **EP 1 006 996 B1**
(45) Date of publication and mention of the grant of the patent: **05.12.2007**
(21) Application number: 98940952.9
(22) Date of filing: 17.08.1998
(51) Int. Cl.: A61K 8/63, A61K 31/565

(54) **USE OF 17-$g(a)-ESTRADIOL FOR THE TREATMENT OF AGED OR SUNDAMAGED SKIN AND/OR SKIN ATROPHY**
VERWENDUNG VON 17-A-ÖSTRADIOL ZUR BEHANDLUNG VON HAUTALTERUNG ODER SONNENSCHÄDIGUNGEN DER HAUT UND/ODER HAUTATROPHIE
UTILISATION DU 17-$g(a)-ESTRADIOL POUR TRAITER LES PEAUX VIEILLIES OU ABIMEES PAR LE SOLEIL ET/OU L'ATROPHIE DE LA PEAU

(30) Priority: 21.08.1997 US 56485 P; 05.08.1998 US 129645; 10.08.1998 US 131549
(43) Date of publication of application: 14.06.2000
(73) Proprietor: JOHNSON & JOHNSON CONSUMER COMPANIES, INC., Skillman, New Jersey 08558-9418 (US)
(72) Inventor: GENDIMENICO, Gerard, J., Neshanic Station, NJ 08853 (US); MEZICK, James, A., East Brunswick, NJ 08816 (US)
(74) Representative: Fisher, Adrian John
(86) International application number: PCT/US1998/017046
(87) International publication number: WO 1999/008654

(56) References cited:
- EP-A- 0 765 663
- WO-A-87/07138
- US-A- 4 390 532
- US-A- 5 656 286
- M. MARTIN-SMITH: "Steroids in Dermatology and Cosmetics" SOAP, PERFUMERY & COSMETICS, vol. 36, 1963, pages 1097-1103, XP002089138 london
- STN, File Supplier, Karlsruhe, DE, XP002089139
- STN, File Supplier, Karlsruhe, DE, XP002089140
- STN, File Supplier, Karlsruhe, DE XP002089141

## Description

### Background of the Invention

Estrogens are hormonal compounds that exert wide-ranging biological effects on target tissues. The major organs affected by estrogens are the reproductive tract, genitals, bone, blood vessels and skin.

The most important natural estrogen is 17-β-estradiol, considered the hormone responsible for most, if not all the normal physiologic processes of this class of compounds. Two other natural estrogens are estrone, a precursor of 17-β-estradiol and estriol, a metabolic product of 17-β-estradiol.

Another estrogen, 17-α-estradiol, is the isomer or epimer of 17-β-estradiol and is biologically much weaker than its β-isomer when given systemically. In animal studies, 17-α-estradiol is reported to possess only 0.4 to 2% of the potency of 17-β-estradiol on reproductive function in rodents. When administered to humans subcutaneously, 17-α-estradiol lacked biological activity on a number of markers of reproductive function. For these reasons, 17-α-estradiol is thought to be a physiologically unimportant estrogen and, accordingly, to have minimal therapeutic value or medicinal use.

The skin is known to respond to some estrogens including 17-β-estradiol. Estrogens stimulate fibroblasts within the dermis and increase the levels of connective tissue matrix molecules. The synthesis of collagen, elastin and non-protein molecules such as hyaluronic acid are elevated when estrogens are administered by systemic or topical routes. Consequently, skin maintains or has an enhanced thickness and integrity. Such skin has a better appearance and is functionally able to resist environmental stresses. Topical treatment of the skin of estrogen-deficient women also showed effacement of wrinkles, increase in water content and improved skin elasticity when treated topically with 17-β-estradiol. In addition, when wounded, skin would be expected to heal more rapidly in the presence of estrogens because fibroblasts would be in an activated state.

Because 17-β-estradiol affects multiple organ systems, it is not desirable to use this chemical in treatment solely of topical skin-related conditions. Rather, it would be more desirable to use an estrogen that is more selective in its action on skin. Although 17-β-estradiol can be administered topically and have local effects on skin, it has the potential to be absorbed into the bloodstream where it can cause undesirable biological responses in non-cutaneous tissues. For example, the delivery of 17-β-estradiol for estrogen replacement therapy is commonly accomplished through sustained release from a device that is applied topically to skin. This method of delivery demonstrates that 17-β-estradiol is ideally suited as a compound to cross the skin barrier and accumulate in the bloodstream. Thus by this route, sufficient blood levels of 17-β-estradiol can be achieved to exert biological activity on all organ systems. Thus, topical administration of 17-β-estradiol is undesirable, although its effects may be beneficial to the skin.

Surprisingly, we have discovered that a relatively unimportant entity which has low potency in biological systems, 17-α-estradiol, may be used as a topical treatment that is more selective in its effects on skin. Our studies show that topical 17-α-estradiol is just as effective as 17-β-estradiol at inducing the synthesis of new connective tissue molecules in the dermis of mouse skin, yet, because of its weak activity as a systemically administered estrogen, 17-α-estradiol is expected to exert insignificant biological responses on non-cutaneous tissues such as the reproductive tract if it is absorbed into the bloodstream after topical application. While the use of 17-α-estradiol has been reported in connection with alopecia, hair loss or hair growth, there has been no data showing that it was effective in the treatment of other skin conditions.

Thus it is an object of our invention to provide a method for treatment of skin conditions where stimulated connective tissue synthesis is beneficial.

It is yet another object of this invention to provide a method for treatment of skin conditions including aged, sun-damaged skin, skin atrophy and for healing of wounds, without undue side effects.

### Summary of the Invention

We have found, surprisingly, that a composition containing a connective tissue synthesizing-effective amount of 17-α-estradiol and a pharmaceutically acceptable carrier can be used in a method of treating the skin which would benefit from connective tissue synthesis by topical application. Unexpectedly, the compositions are capable of generating, connective tissue synthesis when applied topically, but do not engender other biological or physiological effects systemically. Furthermore, we have also found that an epidermal proliferation enhancing-effective amount of 17-α-estradiol and a pharmaceutically acceptable carrier can be used in a method of treating the skin which would benefit from enhanced epidermal proliferation, such as in the treatment of acne and other such skin conditions.

### Detailed Description of Preferred Embodiments

As claimed in claims 1 and 9, this invention relates to the use of 17-α-estradiol as a topical treatment of skin which requires the stimulation of connective tissue synthesis, such as skin which is aged or sun-damaged and/or enhancement of epidermal proliferation, such as skin which is affected by acne or other conditions.

More particularly, this invention relates to methods of using a composition for treatment of aged, sun-damaged skin, skin atrophy, acne and for healing of wounds and possibly for prevention of scarring due to trauma to the skin, *inter alia*. The estrogen 17-α-estradiol is a compound of the following structure:

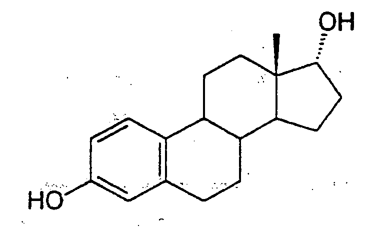

Compositions used in the present invention preferably contain 17-α-estradiol in an effective amount to stimulate connective tissue synthesis and/or enhance epidermal proliferation and/or generally affect skin-related growth processes. Preferably, the compositions of this invention contain from about 0.001% to about 5%, more preferably from about 0.01% to about 2% and most preferably from about 0.1% to about 1% by weight of the active ingredient, 17-α-estradiol.

The compounds which are active in the methods of this invention may be delivered topically by any means known to those of skill in the art. If the delivery parameters of the topically active pharmaceutical or cosmetic agent so require, the topically active composition may preferably be further composed of a pharmaceutically or cosmetically acceptable vehicle capable of functioning as a delivery system to enable the penetration of the topically active agent into the skin. Thus, the topically active composition may be formulated in the form of a liquid, gel or cream or any other conventional pharmaceutically or cosmetically acceptable Vehicle.

Preferably, the compositions are applied to the skin over a period of time sufficient to affect skin-related processes, e.g., stimulate connective tissue synthesis or enhance epidermal proliferation. Generally, the effects of the application and use of the compositions become visible after approximately four to about twenty-four weeks of treatment. It is preferable to apply the compositions in an effective amount once or twice per day.

The compositions may contain, in addition to 17-α-estradiol, other dermatologically active compounds and materials selected from sunscreen ingredients, alpha hydroxy acids, retinoids, beta hydroxy acids, antiinflammatories, antibacterials, antifungals and antioxidants. Such other active ingredients may be combined with 17-α-estradiol to treat and/or present particular dermatological conditions such as erythema, acne, photodamage, psoriasis and others known to those of ordinary skill in the art.

The compositions may be formulated as creams, oils, gels, emulsions, ointments or other forms known to those of ordinary skill in the art.

The stimulatory activity of 17-α-estradiol on connective tissue synthesis in the dermis may be demonstrated by the following two methods. In one method, a deficit in connective tissue synthesis is induced by long-term, exposure of mouse skin to ultraviolet (UV) radiation. This model has been used to demonstrate the reparative effects of retinoids on connective tissue synthesis after cessation of UV exposure. The repair can be visualized as a region of new connective tissue which forms as a subepidermal band in the papillary dermis after topical retinoid treatment (Kligman LH, Chapter 10: Animal Models of Photodamage and Its Treatment, pp. 136-156, In: Photodamage, edited by B. A. Gilchrest, 1995, Blackwell Science, Cambridge, MA). The old malformed elastotic tissue, produced during UV exposure, is compressed by the retinoid-induced new connective tissue zone.

In the second method, topical estrogens increase the thickness of the entire skin by enhancing its water content. This is due to estrogen-stimulated increases in hyaluronic acid, which is a biomolecule that has an avid water binding capacity (Grosman et al. Acta Pharmacol. Toxicol., 1971, Vol. 30, pp. 458-464).

Connective tissue photodamage was induced in albino hairless mice (Skh-1) (six to eight weeks old) by exposure of dorsal trunk skin to UV from FS40 fluorescent tubes, three days per week (Monday, Wednesday, Friday) for ten weeks. The UV dose started with one minimal erythemal dose (MED) and was increased gradually to 4.5 MEDs by week 5 and then continued at 4.5 MEDs for five more weeks. At the beginning of week 11, mice were treated topically with 0.1 mL test material on the dorsal trunk skin, three times per week (Monday, Wednesday, Friday) for 6 to 10 weeks. At the end of treatment, dorsal skin was excised and placed in 10% buffered formalin. The specimens were embedded, sectioned and stained with Luna's aldehyde fuchsin for elastic fibers. The dermal repair zone, defined as the area from the epidermal-dermal junction to the top of the compressed elastotic material, was quantified by measuring the depth of the zones with an image analyzer.

Estrogen-induced thickening of the skin was assessed in albino hairless mice (Skh-1). The skin is topically treated for 3 days with 0.1 mL test material. The next day after the last dose, double skin-fold thickness is measured with an engineer's vernier calipers.

The following examples serve as illustrations of the compositions of this invention, however, they do not limit the scope of the invention described herein.

### Example 1

Albino hairless mice were treated for 10 weeks as set forth above and the repair zone depth was measured.

| Treatment | Dose (%) | Repair Zone Depth |
|---|---|---|
| | | (µm) ± SE |
| Vehicle | -- | 24.9 ± 0.8 |
| 17-α-estradiol | 0.50 | 71.8 ± 3.0 |
| 17-β-estradiol | 0.01 | 57.4 ± 6.1 |
| All-*trans*-retinoic- acid | 0.03 | 72.0 ± 2.6 |

The results show that 17-α-Estradiol at 0.50% stimulates connective tissue repair more than 17-β-Estradiol and equal to all-trans-retinoic acid, the latter being well known for its beneficial effects on aged or sun-damaged skin.

### Example 2

This example shows the dose response relationships of 17-α-Estradiol when used for 6 to 10 weeks.

| Treatment | Dose (%) | Repair Zone Depth (µm) ± SE | |
|---|---|---|---|
| | | 6 Weeks | 10 Weeks |
| Vehicle | -- | 24.5 ± 1.0 | 25.2 + 1.7 |
| 17-α-Estradiol | 0.05 | -- | 36.8 ± 2.4 |
| 17-α-Estradiol | 0.1 | -- | 44.3 ± 2.3 |
| 17-α-Estradiol | 0.5 | 44.6 ± 2.7 | 51.6 + 3.5 |
| 17-β-Estradiol | 0.1 | 46.3 ± 0.9 | 38.7 ± 2.5 |
| All-trans retinoic acid | 0.03 | 44.5 ± 3.9 | 59.0 ± 2.3 |

The results show a response dependent upon the dose of the compound as well as the effect of treatment time. Again, 17-α-Estradiol is equivalent in its effects on connective tissue repair compared to 17-β-Estradiol and all-trans-retinoic acid after either six or ten weeks of treatment.

### Example 3

This example compares the effects of 17-α-Estradiol and 17-β-Estradiol at increasing concentrations compared to the effect of all-*trans*-retinoic acid. Treatments were for 10 weeks.

| Treatment | Dose | Repair Zone Depth |
|---|---|---|
| | (%) | (µm) ± SE |
| Vehicle | -- | 28.0 ± 3.1 |
| 17-α-Estradiol | 0.05 | 34.3 ± 3.7 |
| 17-α-Estradiol | 0.01 | 49.4 ± 3.7 |
| 17-α-Estradiol | 0.5 | 62.4 ± 4.2 |
| 17-β-Estradiol | 0.005 | 45.2 ± 3.1 |
| 17-β-Estradiol | 0.01 | 53.4 ± 2.1 |
| 17-β-Estradiol | 0.1 | 68.2 ± 6.0 |
| All-*trans*-retinoic acid | 0.03 | 72.0 + 3.3 |

This shows a dose dependent response of 17-α-Estradiol and 17-β-Estradiol. While 17-β-Estradiol is more potent, 17-α-Estradiol also shows identical therapeutic effect.

### Example 4

This example compares the effects of 17-α-Estradiol and 17-β-Estradiol on skin thickening after 3 days of topical treatment.

| Treatment | Dose (%) | Skin-Fold Thickness |
|---|---|---|
| | | (mm) ± SE |
| Vehicle | -- | 1.12 ± 0.05 |
| 17-α-Estradiol | 0.01 | 1.16 ± 0.07 |
| 17-α-Estradiol | 0.1 | 1.43 ± 0.06 |
| 17-α-Estradiol | 0.5 | 1.52 ± 0.14 |
| 17-β-Estradiol | 0.001 | 1.25 ± 0.08 |
| 17-β-Estradiol | 0.01 | 1.62 ± 0.11 |
| 17-β-Estradiol | 0.1 | 1.50 ± 0.13 |

This demonstrates that 17-α-Estradiol is as effective as 17-β-Estradiol in its effects on skin thickening due to water retention mediated by increased amounts of hyaluronic acid.

All of these examples show the unexpected topical activity of 17-α-Estradiol on skin connective tissue. Furthermore, since 17-α-Estradiol does not show other estrogenic responses, it has none of the disadvantages of other known estrogens such as 17-β-Estradiol.

### Example 5

This example shows the dose response relationships of 17-α-Estradiol on thickness of the epidermis when used for 10 weeks.

| Treatment | Dose (%) | Epidermal Thickness |
|---|---|---|
| | | (µm) ± SE |
| Vehicle | -- | 26.6 ± 1.9 |
| 17-α-Estradiol | 0.05 | 29.3 ± 0.3 |
| 17-α-Estradiol | 0.1 | 37.3 ± 1.8 |
| 17-α-Estradiol | 0.5 | 42.9 ± 3.8 |
| 17-β-Estradiol | 0.005 | 32.0 ± 1.2 |
| 17-β-Estradiol | 0.01 | 28.3 ± 1.8 |
| 17-β-Estradiol | 0.1 | 48.1 ± 2.9 |
| All-*trans*-retinoic acid | 0.03 | 52.8 ± 3.6 |

These results show that 17-α-Estradiol and 17-β-Estradiol act like the retinoid, all-trans-retinoic acid by increasing the thickness of the epidermis. Thus 17-α-Estradiol would be beneficial for skin conditions where the epidermis is atrophied, either due to intrinsic aging of the skin or in pathological conditions caused by excessive sun exposure, wounding, etc. Enhanced epidermal proliferation would also be of value in conditions like acne where stimulation of the follicular epithelia would have a comedolytic effect aiding the expulsion of hyperkeratinized material.

## Claims

1. The use of 17-α-estradiol in the manufacture of a composition for wound healing or for the therapeutic treatment of a mammalian skin condition selected from, undamaged skin, skin atrophy and acne, wherein said composition does not contain any further dermatologically active compounds or said composition contains further dermatologically active compounds, provided that said further dermatologically active compounds are sunscreen ingredients, alpha ingredients, alpha hydroxy acids, retinoids, beta hydroxy acids, antiinflammatories, antibacterials, antifungals or antioxidants.

2. The use according to claim 1, wherein the composition comprises from 0.001% to 5% of 17-α-estradiol.

3. The use according to claim 1, wherein the composition comprises from 0.05% to 1% of 17-α-estradiol.

4. The use according to claim 1, wherein said composition comprises a pharmaceutically acceptable carrier suitable for topical application to mammalian skin.

5. The use according to claim 4, wherein said composition is for treatment of a chronic skin condition.

6. The use according to claim 5, wherein the composition is for treatment of the skin for a period of at least 3 months comprising application of the composition from once a week to twice a day.

7. The use according to claim 6, wherein the composition comprises another agent selected from the group consisting of a safe and effective amount of a sunscreening agent, and an antioxidant.

8. The use according to claim 1, wherein said composition is for oral administration.

9. A non-therapeutic method of cosmetic treatment of mammalian skin conditions selected from aged skin, and skin atrophy, comprising treating skin with a safe and effective amount of a composition comprising:
(a) a safe and effective amount of 17-α-estradiol; and
(b) a cosmetically acceptable carrier,
wherein said composition does not contain any further dermatologically active compounds or said composition contains further dermatologically active compounds, provided that said further dermatologically active compounds are sunscreen ingredients, alpha hydroxy acids, retinoids, beta hydroxy acids, or antioxidants.

## Patentansprüche

1. Verwendung eines 17-α-Östradiol zur Herstellung einer Zusammensetzung zur Wundheilung oder zur therapeutischen Behandlung eines Zustands einer Säugetierhaut, ausgewählt aus sonnengeschädigter Haut, Hautatrophie und Akne, wobei die Zusammensetzung keine weiteren dermatologisch aktiven Verbindungen enthält oder die Zusammensetzung weitere dermatologisch aktive Verbindungen enthält, vorausgesetzt, daß die weiteren dermatologisch aktiven Verbindungen Sonnenschutzinhaltsstoffe, alpha-Hydroxysäuren, Retinoide, beta-Hydroxysäuren, antientzündliche Substanzen, antibakterielle Substanzen, Anti-Pilzsubstanzen oder Antioxidantien sind.

2. Verwendung nach Anspruch 1, wobei die Zusammensetzung von 0.001% bis 5% 17-α-Östradiol umaßt.

3. Verwendung nach Anspruch 1, wobei die Zusammensetzung von 0,05% bis 1% 17-α-Östradiol umfaßt.

4. Verwendung nach Anspruch 1, wobei die Zusammensetzung einen pharmazeutisch akzeptablen Träger umfaßt, der für eine topische Anwendung auf Säugetierhaut geeignet ist.

5. Verwendung nach Anspruch 4, wobei die Zusammensetzung zur Behandlung eines chronischen Hautzustandes dient.

6. Verwendung nach Anspruch 5, wobei die Zusammensetzung zur Behandlung der Haut für eine Periode von wenigstens 3 Monaten dient, umfassend die Anwendung der Zusammensetzung von einmal pro Woche bis zu zweimal am Tag.

7. Verwendung nach Anspruch 6, wobei die Zusammensetzung ein anderes Mittel umfaßt, ausgewählt aus der Gruppe, bestehend aus einer sicheren und wirksamen Menge eines Sonnenschutzmittels und einem Antioxidans.

8. Verwendung nach Anspruch 1, wobei die Zusammensetzung zur oralen Verabreichung dient.

9. Nicht-therapeutisches Verfahren zur kosmetischen Behandlung von Zuständen einer Säugetierhaut, ausgewählt aus gealterter Haut und Hautatrophie, umfassend das Behandeln der Haut mit einer sicheren und wirksamen Menge einer Zusammensetzung, umfassend:
(a) eine sichere und wirksame Menge an 17-α-Östradiol; und
(b) einen kosmetisch akzeptablen Träger,
wobei die Zusammensetzung keine weiteren dermatologisch aktiven Verbindungen enthält oder die Zusammensetzung weitere dermatologisch aktive Verbindungen enthält, vorausgesetzt, daß die weiteren dermatologisch aktiven Verbindungen Sonnenschutzinhaltsstoffe, alpha-Hydroxysäuren, Retinoide, beta-Hydroxysäuren oder Antioxidantien sind.

## Revendications

1. Utilisation de 17-α-estradiol dans la fabrication d'une composition cicatrisante ou pour le traitement thérapeutique d'une condition de la peau des mammifères choisie parmi la peau abîmée par le soleil, l'atrophie cutanée et l'acné, où ladite composition ne contient aucun autre composé ayant une activité dermatologique, ou contient d'autres composés ayant une activité dermatologique à condition que lesdits autres composés ayant une activité dermatologique soient des ingrédients d'écran solaire, des acides alpha-hydroxy, des rétinoïdes, des acides bêta-hydroxy, des anti-inflammatoires, des antibactériens, des antifongiques ou des antioxydants.

2. Utilisation selon la revendication 1, dans laquelle la composition comprend de 0,001 % à 5 % de 17-α-estradiol.

3. Utilisation selon la revendication 1, dans laquelle la composition comprend de 0,05 % à 1 % de 17-α-estradiol.

4. Utilisation selon la revendication 1, dans laquelle ladite composition comprend un support pharmaceutique acceptable pour une application topique à la peau des mammifères.

5. Utilisation selon la revendication 4, dans laquelle ladite composition est une composition pour le traitement d'une condition cutanée chronique.

6. Utilisation selon la revendication 5, dans laquelle la composition est une composition pour le traitement de la peau sur une durée d'au moins 3 mois, consistant en l'application de la composition d'une fois par semaine à deux fois par jour.

7. Utilisation selon la revendication 6, dans laquelle la composition comprend un autre agent choisi dans le groupe consistant en une quantité sûre et efficace d'un agent d'écran solaire et d'un antioxydant.

8. Utilisation selon la revendication 1, dans laquelle ladite composition est destinée à une administration orale.

9. Méthode de traitement cosmétique non thérapeutique de conditions de la peau des mammifères choisies parmi la peau âgée et l'atrophie cutanée, comprenant le traitement de la peau avec une quantité sûre et efficace d'une composition comprenant :
(a) une quantité sûre et efficace de 17-α-estradiol ; et
(b) un support cosmétique acceptable ;
dans laquelle ladite composition ne contient aucun autre composé ayant une activité dermatologique ou contient d'autres composés ayant une activité dermatologique à condition que lesdits autres composés ayant une activité dermatologique soient des ingrédients d'écran solaire, des acides alpha-hydroxy, des rétinoïdes, des acides bêta-hydroxy ou des antioxydants.
